# EUROPEAN PATENT APPLICATION

(11) **EP 4 681 750 A1**
(43) Date of publication of application: **21.01.2026**
(21) Application number: 24306215.5
(22) Date of filing: 18.07.2024
(51) Int. Cl.: A61M 5/32

(54) **A SAFETY DEVICE FOR A SYRINGE**

(71) Applicant: Becton Dickinson France, 38800 Le Pont-de-Claix (FR)
(72) Inventor: FIARD, Michael, 38320 Eybens (FR); CARREL, Franck, 38220 Saint Jean de Vaulx (FR); BESSON, Nicolas, 38650 TREFFORT (FR); DELOBELLE, Vincent, 38420 DOMENE (FR)
(74) Representative: Germain Maureau

(57) **Abstract**

The safety device (101) comprises:
- a body (20) configured to receive a syringe (10);
- a guard (30) movable relative to the body between an injection position in which the syringe needle (14) extends beyond the distal end of the guard, and a safety position in which the guard covers the needle;
- a skin contact member (50) which is arranged at and secured to the distal end portion of one element among the body (20) and the guard (30), said element being called the support element.

The skin contact member (50) comprises a collar (51) having a distal face (52) that extends substantially in a plane orthogonal to the safety device axis (105), said distal face being configured to form a contact surface with the skin and to be located proximally from the needle tip in the injection position; The collar has an outer edge and an inner edge defining a hole for the needle (14) to pass through.

## Description

The invention relates to a safety device for a syringe, and to an injection system comprising such a safety device and a syringe received in the body.

More specifically, the invention relates to a safety device which comprises a body configured to receive the syringe and a guard movable relative to the body between an injection position in which the needle extends beyond the distal end of the guard, and a safety position in which the guard covers the needle.

This injection system is advantageous in that it ensures the user does not prick himself after injection.

However, the use of such an injection system is not always easy, as the appropriate gestures to prick the patient at the appropriate skin depth is complex for non-trained people. In practice, the user needs to grasp the patient's skin to stretch it, such an action being called the skin pinch. Then, when pricking the patient, the needle must be oriented at a predetermined angle with respect to the skin surface at the injection site, for example around 45°. This ensures the needle reaches the appropriate depth, i.e. the targeted tissues (for example subcutaneous or muscles area). Generally, a nurse needs to be present to perform such an injection.

Existing solutions to this problem involve the use of add-ons in the form of large devices which house the whole injection system. In addition to being technically complex, these solutions drastically increase the environmental impact per injection.

An object of the invention is to provide an injection system which is easier to use appropriately than known devices, thus making it possible for patients to prick themselves rather than requiring the help of a nurse coming to their home. Another object is to reach this result with a limited environmental impact.

For that purpose, and according to a first aspect, the invention relates to a safety device for a syringe having a needle, the safety device having an axis and comprising:
- a body configured to receive the syringe, the body having a distal opening;
- a guard having a distal opening, the guard being movable relative to the body between an injection position in which the needle extends beyond the distal end of the guard, and a safety position in which the guard covers the needle;
wherein the safety device further comprises a skin contact member which is arranged at and secured to the distal end portion of one element among the body and the guard, said element being called the support element. Said skin contact member comprises a collar having a distal face that extends substantially in a plane orthogonal to the safety device axis, said distal face being configured to form a contact surface with the skin and to be located proximally from the needle tip in the injection position. Also, the collar has an outer edge and an inner edge defining a hole for the needle to pass through.

In this application, the distal end of a component or apparatus must be understood as meaning the end furthest from the hand of the user and the proximal end must be understood as meaning the end closest to the hand of the user, with reference to the injection system including said component or apparatus. As such, in this application, the distal direction must be understood as the direction of injection with reference to the injection system, and the proximal direction is the opposite direction.

Because of the arrangement of the collar distal face, the safety device is naturally placed by a user with its axis orthogonal to the skin during injection. Furthermore, the needle exposure length, i.e. the length of the part of the needle that extends distally beyond the safety device, in the injection position, is set by design.

As a result, even a non-experienced user can prick himself and achieve the desired result. Indeed, on the one hand, no skin pinch being necessary, no technical action is to be performed. On the other hand, the correct positioning of the safety device relative to the skin is intuitive owing to the flat contact surface of the collar, and such a correct positioning automatically implies a correct needle depth. This ensures that the right tissues are reached by the needle, and thus that the injection process is carried out appropriately.

The skin contact member layout has further advantages.

First of all, the skin contact member being arranged at the distal end portion of the body or the guard, it has small dimensions as compared to the whole safety device. This results in notable savings on raw materials and in low environmental impact for a function that is generally done through a complex add on system.

In addition, the skin contact member is secured to the distal end portion of the body or the guard.

Owing to these dispositions, the skin contact member can be quite easily implemented either on current safety devices as an add-on of small dimensions, or on current manufacturing tools to provide an upgraded safety device. Besides, being located at - preferably confined - and secured to the distal end portion of the body or the guard, the skin contact member has no impact on features located on other parts of the body or the shell, such as features maintaining the guard in the injection position or causing the guard to move to the safety position.

Also, it has to be noted that the skin contact member is secured to one element among the body and the guard, and therefore the modification brought by the invention concerns only one element. Thus, the advantageous result can be achieved with a low impact and at a low cost.

Several implementations are possible provided the movement of the guard with respect to the body remains possible even with the presence of the collar.

Thus, with the guard being arranged outside the body i.e. receiving the body, the support element can be the guard. In a variant, the support element can be the body, a sufficient radial gap then being necessary between the body and the guard to accommodate the collar.

And with the guard being inside the body, i.e. being received in the body, the support element can be the body or the guard.

In practice, the support element can be the one among the body and the guard which extends further distally in the injection position.

In an implementation, the syringe is housed in the body, with the syringe barrel having no freedom of movement relative to the body, whether in the axial direction or in rotation about the safety device axis.

According to different embodiments, the body can be housed in the guard or the guard can be housed in the body.

The collar outer edge may be at least partially located radially further from the axis than the support element outer face. In other words, in such a case, at least part of the collar extends radially beyond the outer surface of the support element, when seen in projection in a plane orthogonal to the axis.

Owing to this arrangement, the surface area of the contact surface with the skin is large enough for the injection system to be appropriately positioned against the skin by the user, and to provide a good stability of the injection system in said position.

The surface area of the contact surface with the skin, which is provided by the collar distal face, may be at least 1,4 or 2 or 4 times the surface area of the support element distal opening.

The collar may have an annular shape, with an inner edge and an outer edge that are circular and concentric. In a variant of such a shape, the outer edge may be rectangular, triangular or ovoid. However, the collar is not necessarily annular; it does not necessarily have a regular or symmetrical shape. The inner and outer edges may not be circular.

In case the collar has a regular shape and its inner and outer edges are both circular, the outer edge diameter may be N times larger than the inner edge diameter, N being comprised between 1,2 and 2, 5 or between 1,3 and 2 or between 1,4 and 1,8.

In case the support element comprises a tubular portion and finger flanges extending laterally from said tubular portion, the outer edge of the collar may extend radially from the axis over a distance that is N' times the maximum distance between the axis and the outer face of the support member tubular portion, N' being at least 1,2 or 1,4 or 1,5 and less than 3 or 2.

The axial length of the skin contact member may be comprised between 4 and 30 %, or 5 and 25 %, or 5 and 10 %, of the axial length of the support element. Thus, the skin contact member extends over a relatively small part of the axial length of the support element. This makes it possible to make material savings.

In an embodiment, the collar has the shape of a plate and has a proximal face that extends substantially in a plane orthogonal to the safety device axis. Then, the distal face of the plate can form the distal face of the collar.

In an embodiment, the skin contact member comprises a cylindrical sleeve. The cylindrical sleeve has an inner diameter which may be substantially identical to the diameter of the support element distal opening. The proximal face of the cylindrical sleeve may be adjacent the support element distal face, or may be in contact with the support element distal face. The collar may protrude radially outwardly from the sleeve distal end. The inner face of the sleeve may form the inner edge of the collar.

According to an embodiment, the skin contact member is a part separate from the support element and secured to the distal end portion of said support element. The skin contact member can be secured to the support element by gluing, welding or heat bonding. Alternatively or in addition, the skin contact member can comprise attaching means for attachment to the support element, such as snap features.

Thus, a skin contact member can be implemented on an existing safety device, without requiring significant changes to existing molds or tools.

The skin contact member can be secured to the support element distal end face.

The skin contact member can comprise a proximal casing having a proximal aperture, the distal end portion of the support element being received in said proximal casing and secured thereto.

Having a skin contact member designed as a separate part makes it possible to mount it on an existing safety device which comprises an extended finger flange secured to a finger flange of the guard. Indeed, such an extended finger flange is generally assembled on the guard from the distal end and moved distally to be secured to its final position. This assembling movement would not be possible with a previously mounted collar that would form an obstacle to the insertion of the extended finger flange.

According to another embodiment, the skin contact member is integral with the support element, for example molded as a single piece with said support element.

In such a case, with the support element being the body, it may be provided that the body has a cylindrical shape and the cylindrical sleeve forms the extension of the cylindrical shape of the body. In other words, the sleeve and the body may form one and the same cylinder.

The skin contact member can comprise contact-promoting means to improve the contact with the patient's skin, at the injection site.

The skin contact member may comprise at least one protrusion which protrudes distally from the distal face of the collar and which is configured to improve the contact between the collar and the skin. The aim of such a protrusion is to prevent slipping, to improve grip and/or to provide a better contact feeling. The protrusions may include discrete domes, radial ribs, etc.

The distal face of the collar of the skin contact member may be at least partially covered with an adhesive. The aim of such an arrangement is to provide better stability.

According to a second aspect, the invention concerns an injection system comprising a safety device as previously described and a syringe received in the body, the syringe having a needle. In the injection position, the needle tip is located proximally from the collar distal face. Owing to the skin contact member, the invention allows controlling the distance over which the needle extends beyond the collar distal face, i.e. the needle exposure length, which ensures an appropriate pricking depth.

For example, the injection system further comprises a needle shield attached to the syringe for covering the needle in the storage position. The injection system can further comprise a cap which is removably mounted on the skin contact member collar around the needle shield, the cap having inner attaching means which are disengaged from the needle shield in the storage position and which can move to engage the needle shield in a first phase of the removal of the cap from the collar, so that in a second phase, the removal of the cap from the collar entails the removal of the needle shield from the syringe. In this implementation, the collar is not only used as a contact surface with the skin, but also as an element to which the cap can be attached.

Possible embodiments of the invention will now be described by way of nonlimiting examples with reference to the appended figures:
Figure 1 is an exploded view of an injection system according to a first embodiment of the invention, the injection system comprising a syringe, a body and a guard having a skin contact member;
Figure 2 shows the injection system in an injection position;
Figure 3 shows the injection system in a safety position;
Figure 4 is a perspective view of the guard and the skin contact member;
Figure 5 is an axial view of the guard from the proximal side;
Figure 6 is a detailed view of a distal end portion of the guard, showing the skin contact member;
Figures 7 to 9 show variants of the skin contact member;
Figure 10 schematically shows a skin contact member about to be secured to the guard;
Figure 11 schematically shows a skin contact member having attaching means for being secured to the guard;
Figure 12 shows a variant of the guard and the skin contact member;
Figure 13 is a longitudinal cross-section view of the injection system according to the variant of figure 12;
Figure 14 is a detailed view of the injection system, further comprising a cap mounted on the skin contact member;
Figure 15 is a perspective view of a needle shield to be attached to the syringe for covering the needle in the storage position;
Figure 16 is a longitudinal cross-section view of the distal end portion of the injection system of figure 14, in the storage position, the cap being disengaged from the needle shield;
Figure 17 is a view similar to figure 16, in a phase of the removal of the cap, the cap engaging the needle shield;
Figure 18 is an exploded view of an injection system according to a second embodiment of the invention, the injection system comprising a syringe, a body and a guard having a skin contact member;
Figures 19 and 20 are longitudinal cross-sections of the injection system in two planes orthogonal to each other, the injection system being in an injection position;
Figures 21 and 22 are views similar to Figures 19 and 20, respectively, the injection system being in a safety position.

The invention concerns an injection system 100 which comprises a safety device 101 and a syringe 10.

The syringe 10 comprises a barrel 11 - preferably having a proximal outer flange 12 - a plunger rod 13 provided with a stopper 17, and a needle 14. A needle shield 70 may further be provided to cover the needle 14 before use, to avoid injuries and contamination. The needle shield 70 may be removably mounted on the distal end of the barrel 11. The plunger rod 13 may further comprise a proximal member 16 pushed by a user's finger to perform injection, which can be disc-shaped.

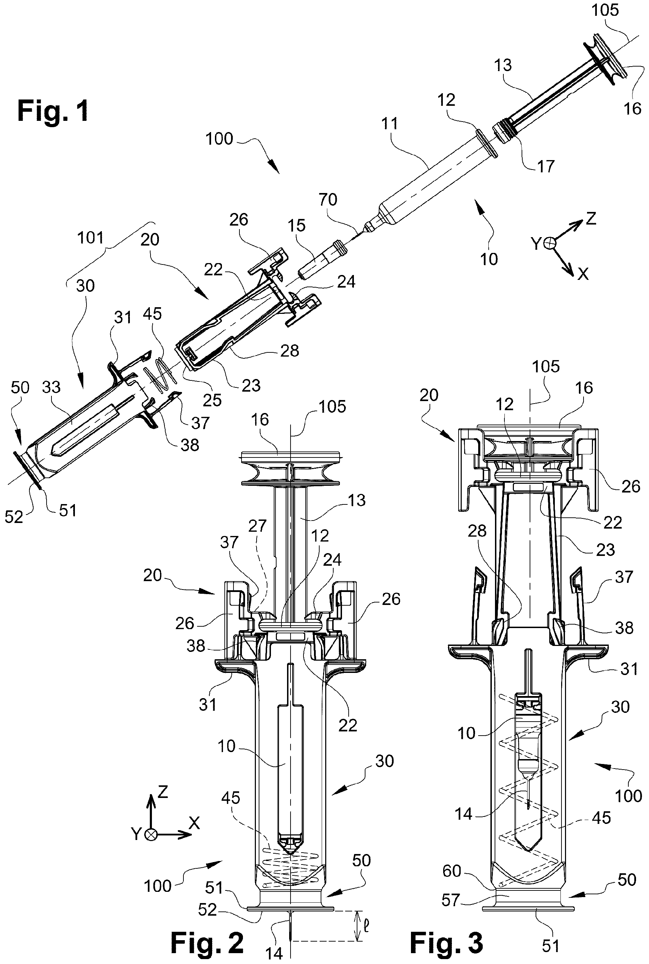

The safety device 101 is configured to receive the syringe 10. The safety device 101 comprises a body 20 configured to receive the syringe 10, and a guard 30. The guard 30 is movable relative to the body 20 between:
- an injection position (figures 2, 19 and 20) in which the needle 14 extends beyond the distal end of the guard 30, through a distal opening 25 of the body 20 and a distal opening 35 of the guard 30;
- and a safety position (figures 3, 21 and 22) in which the guard 30 covers the needle 14.

By "injection position" is meant the position in which the injection system 100 is ready to perform injection. The safety position is the position of the injection system 100 once injection is completed and the system has been removed from the injection site. It has to be noted that the injection system may also be in a storage position, which corresponds to an initial position, before use, and which may be different from the injection position.

The safety device 101 preferably comprises finger flanges that extend laterally from the body 20 or from the guard 30, preferably adjacent to a proximal end thereof.

The safety device 101 has an axis 105. Preferably, the injection system 100 and its constitutive parts have the same axis 105. Longitudinal direction Z is defined as the direction of axis 105. The terms "distally" and "proximally" are used with reference to said longitudinal direction Z. Lateral direction X is defined as a direction orthogonal to axis 105 and as being the general direction along which the finger flanges of the safety device 101 extend. The transverse direction Y is defined as the direction orthogonal to the longitudinal direction Z and to the lateral direction X.

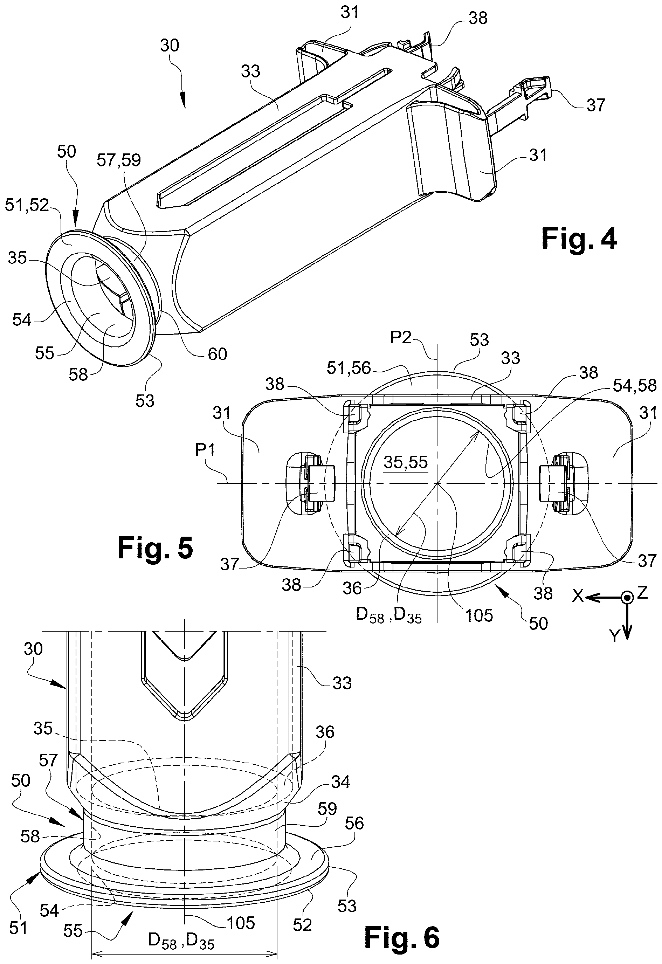

The injection system 100, or at least part of its constitutive parts, may have one or two axes of symmetry, namely a lateral plane P1 parallel to (X,Z) and/or a transverse plane P2 parallel to (Y,Z), as shown on figure 5.

According to the invention, the safety device 101 further comprises a skin contact member 50 which is arranged at and secured to the distal end portion of the guard 30 or of the body 20, as will be described in more detail.

A first embodiment of the invention is illustrated in figures 1 to 17.

The injection system 100 according to this first embodiment can be at least partially similar to the system described in WO 2013/159059, the disclosure of which is hereby incorporated by reference in its entirety.

The body 20 comprises a tubular portion 23 having a distal opening which forms the distal opening 25 of the body 20. The syringe 10 is received in the tubular portion 23 and is secured in the mounted position by appropriate means, such as fasteners 24 projecting inwardly at the proximal part of the body 20. The flange 12 of the barrel 11 may be snapped between said fasteners 24 and a wall 22 of the body 20 which extends orthogonally to axis 105 and which is located distally from the fasteners 24.

The body 20 may also comprise two casings 26 which are arranged on either side of axis 105 along direction X. Each casing 26 is open at its distal end and at its proximal end. Between each casing 26 and the axis 105 is arranged an abutment face 27 which is orthogonal to axis 105 and faces proximally.

Furthermore, the tubular portion 23 of the body 20 may comprise at least one notch 28, and preferably two opposed notches 28, the function of which will be explained later.

The guard 30 preferably comprises a tubular portion 33 having a distal opening which forms the distal opening 35 of the guard 30. The guard 30 is arranged outside the body 20 and receives the body 20. The guard 30 may comprise finger flanges 31 which extend laterally adjacent to a proximal end of the guard 30.

The guard 30 may comprise at least one clamp 37, and preferably two clamps 37, that extends proximally from the proximal end portion of the tubular portion 33, for example from one finger flange 31. Besides, the guard 30 may also comprise at least one lug 38, and preferably four lugs 38, which extends proximally from the proximal end portion of the tubular portion 33, for example from the finger flanges 31, and which is shorter than the clamp 37.

The safety device 101 may further be provided with an extended finger flange (not shown) secured to a finger flange 31 of the guard 30. Such an extended finger flange is described in WO 2011/140144, the disclosure of which is hereby incorporated by reference in its entirety.

A spring 45 is mounted in the guard 30, between the guard 30 and the body 20. The spring 45 biases the body 20 away from the guard 30 in the proximal direction.

In the injection position, as shown in figure 2, the tubular portion 23 of the body 20 is substantially fully located inside the guard 30. The body 20 is maintained in position relative to the guard 30, against the action of the spring 45, for example by means of the clamps 37, each clamp 37 being inserted in a casing 26 and cooperating with an abutment face 27. In this injection position, the needle 14 extends beyond the distal end of the safety device 101.

When injection has been completed, the clamps 37 or other retaining member is released. This can be achieved by the action of the plunger rod 13, for example of the proximal member 16, which can cause the clamps 37 to deflect outwardly and thus to disengage from the abutment faces 27.

As a result, when the injection system 100 has been removed from the patient's skin, and/or when the user stops pressing the plunger rod 13 against the finger flanges 31 of the safety device 101, the spring 45 causes the guard 30 to move distally relative to the body 20, thereby covering at least part of the needle 14. The injection system 100 is then in the safety position, as shown in figure 3. The guard 30 can be maintained in this position relative to the body 20 by the lugs 38 engaged in the notches 28, to prevent the needle 14 from being uncovered again.

According to this first embodiment of the invention, the safety device 101 further comprises a skin contact member 50 which is arranged at and secured to the distal end portion of the guard 30.

The skin contact member 50 comprises a collar 51 which has a distal face 52 that extends substantially in a plane orthogonal to the safety device axis 105. Said distal face 52 is configured to form a contact surface with the patient's skin, at the injection site. The collar 51 has an outer edge 53 and an inner edge 54 which defines a hole 55. In the injection position, the needle 14 passes through the hole 55, and the needle tip is located distally from the collar distal face 52, as can be seen in figure 2.

The collar 51 can have the shape of a plate, and thus can have a proximal face 56 that also extends substantially in a plane orthogonal to the safety device axis 105.

The skin contact member 50 may further comprise a cylindrical sleeve 57 having an inner face 58, an outer face 59, a proximal face 60 and a distal face 61. The collar 51 may protrude radially outwardly from the sleeve distal end, the collar distal face 52 preferably being level with the sleeve distal face 61.

In an embodiment, the sleeve 57 extends in the continuity of the guard 30. In particular, the cylindrical sleeve 57 may have an inner diameter D58 which is substantially identical to the diameter D35 of the distal opening 35 of the guard 30. Moreover, the sleeve proximal face 60 is preferably adjacent or in contact with the guard distal face.

In order to ensure that the contact surface between the skin contact member 50 and the skin has a sufficient area, at least part of the collar 51 preferably extends radially beyond the tubular portion 33 of the guard 30. In other words, the collar outer edge 53 may at least be partially located radially further from the axis 105 than the outer face of the guard tubular portion 33, as can be seen in figure 5 for example. However, other embodiments can be contemplated to reply to specific requirements, such as space requirements.

The skin contact member 50 is designed, given the dimensions of the guard 30, body 20 and syringe 10, and given the predetermined axial position of the body 20 relative to the guard 30 in the injection position, so as to set the needle exposure length I in said position. As shown in figure 2, the needle exposure length I is the length of the part of the needle that extends distally beyond the safety device 100, i.e. beyond the collar 51. As a result, in combination with the fact that the transverse distal face 52 of the skin contact member 50 promotes, if not ensures, an orthogonal positioning of the injection system 100 with respect to the injection site, the invention guarantees any user that the injection system 100 is appropriately placed and that the correct needle depth is achieved.

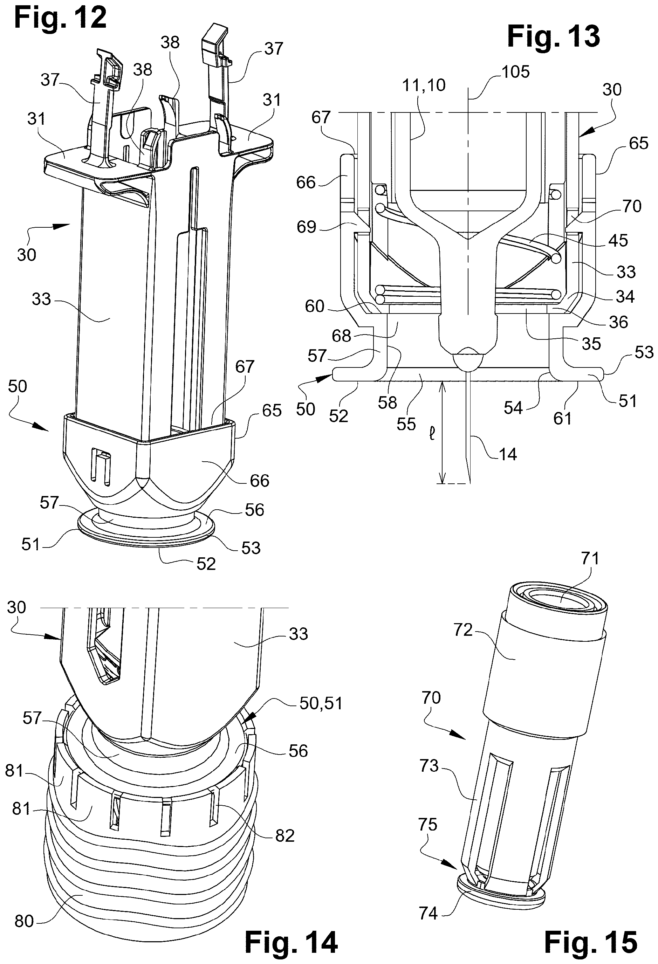

As can be seen in particular in figures 6 and 13, the distal end portion of the guard 30 may comprise, from and beyond the tubular portion 33, a tapered portion 34 which converges distally towards the axis 105, and a ring portion 36 which extends in a plane orthogonal to the axis 105, from the distal end of the tapered portion 34 towards the axis 105. The free end of the ring portion 36 defines the distal opening 35 of the guard 30. The radial dimension of the ring portion 36 may be substantially identical to the thickness of the sleeve 57 of the skin contact member 50, i.e. the distance between the inner face 58 and the outer face 59.

The skin contact member 50 may be secured to the guard 30 so that the sleeve 57 is substantially in the extension of the ring portion 36, in the longitudinal direction and distally. For example, the proximal face 60 of the sleeve 57 may face or may be in contact with the distal face of the guard ring portion 36.

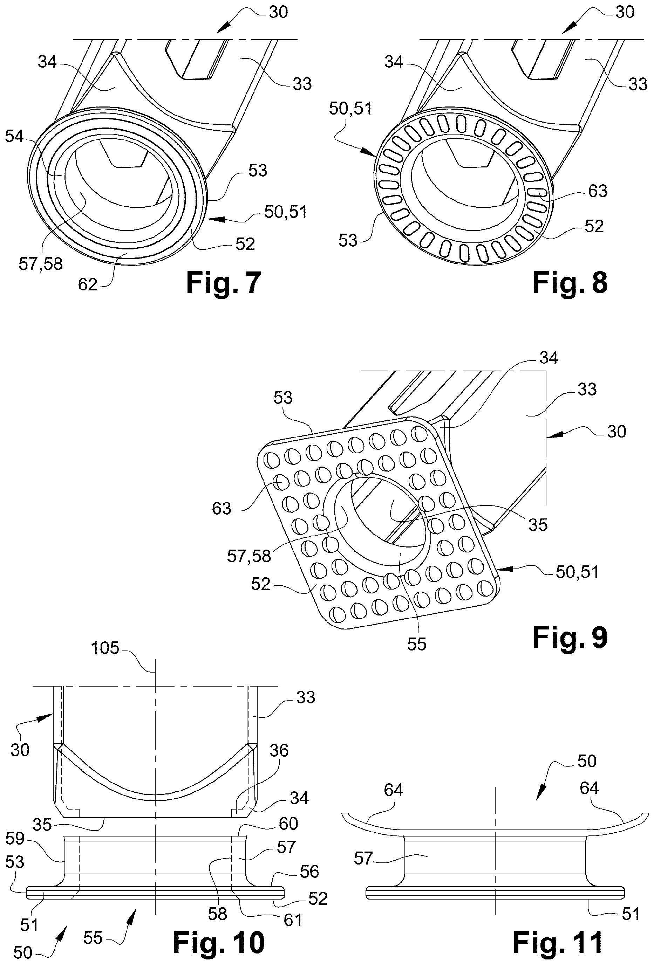

In order to improve the contact between the safety device 101 and the skin, the distal face 52 of the collar 51 of the skin contact member 50 may be at least partially covered with an adhesive 62, as schematically illustrated in figure 7. In a variant, the skin contact member 50 may comprise at least one protrusion 63 which protrudes distally from the distal face 52 of the collar 51. Such protrusions 63 may form ribs, for example arranged radially (figure 8) or may form discrete domes or similar (figure 9).

The collar 51 may be ring-shaped, i.e. may have an outer edge 53 having a circular shape. However, such an arrangement is not to be considered as limiting. Other shapes can be contemplated. For example, as shown in figure 9, the collar outer edge 53 can have a rectangular or square shape.

In the embodiment shown in figures 1 to 6, the skin contact member 50 forms a single piece with the guard 30.

In a first variant, the skin contact member 50 is made as a single piece with the guard 30, for example by molding. In a second variant, the skin contact member 50 is made as a part separate from the guard 30, and secured to the guard 30, at the distal end portion thereof, before being provided to the user. For example, as shown in figure 10, the proximal face 60 of the sleeve 57 of the skin contact member 50 can be secured to the guard distal end face, which can be the distal face of the guard ring portion 36. The assembly can be obtained by any appropriate method such as gluing, welding or heat bonding.

In both variants, the axial length of the skin contact member 50 can be comprised between 4 and 15 %, or between 5 and 10 % of the axial length of the guard tubular portion 33.

In the embodiment shown in figures 11 to 13, the skin contact member 50 is a part separate from the guard 30 which is attached to the distal end portion of the guard 30. For that purpose, as schematically illustrated in figure 11, the skin contact member 50 is provided with attaching means 64 for being secured to the guard 30. These attaching means may allow creating a larger contact surface with the guard 30 to improve the mechanical connection, as compared with the embodiment shown in figure 10. In the embodiment of figures 11 to 13, although the skin contact member 50 and guard 30 are secured to one another, they remain distinct parts.

Figures 12 and 13 show an example of such an arrangement, in which the skin contact member 50 comprises a proximal casing 65. The proximal casing 65 has a peripheral wall 66, a proximal aperture 67 and a distal aperture 68, and is configured to receive the distal end portion of the guard 30. For that purpose, the proximal casing 65 preferably has a shape that matches the outer shape of the distal end portion of the guard 30. The sleeve 57 of the skin contact member 50 protrudes from the distal end of the proximal casing 65, around the distal aperture 68, as can be seen in figure 13.

Furthermore, the proximal casing 65 is secured to the distal end portion of the guard 30. To that end, at least one snap tab 69 may be arranged in the proximal casing peripheral wall 66, said snap tab 69 being configured to cooperate with a corresponding hole 70 provided in the guard tubular portion 33. The locations and dimensions of the snap tab 69 and hole 70 are determined so as to set the needle exposure length I in the injection position.

Such an arrangement can be implemented on an existing guard 30 without changing its features, except for the provision of the hole(s) 70.

With this arrangement, the axial length of the skin contact member 50 can be comprised between 15 and 30%, or between 20 and 30% of the axial length of the guard tubular portion 33.

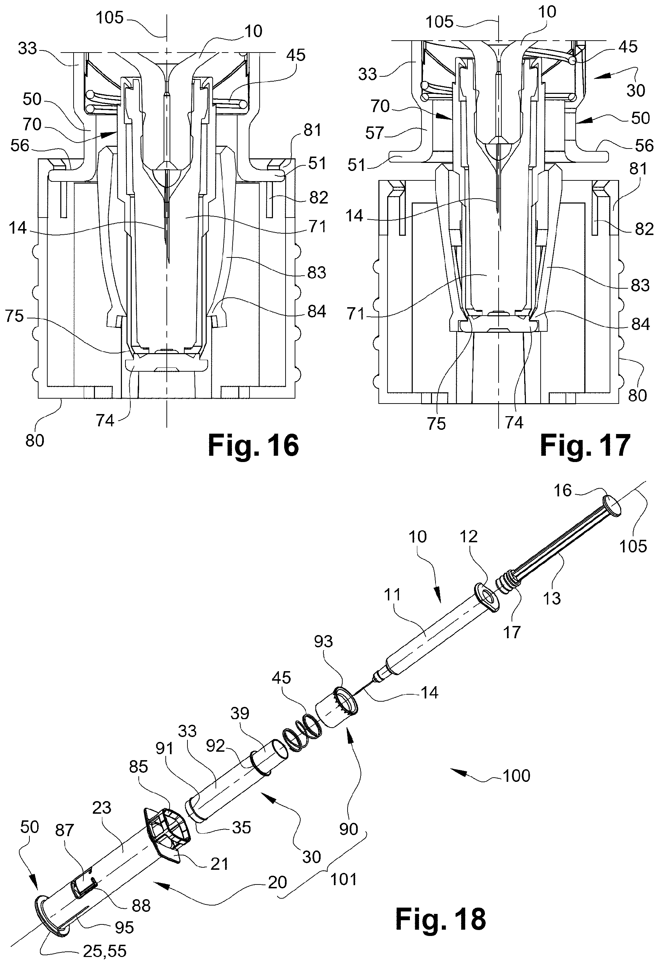

The injection system 100 may further comprise a needle shield 70 which is attached to the syringe 10 for covering the needle 14 in the storage position, as shown in figure 16.

As illustrated in figure 15, the needle shield 70 may include an isolator portion 71 that is made of flexible material and into which the needle tip is poked in the storage position, to keep the content of the syringe 10 sterile. The needle shield 70 may also include an outer structure 72 which is made of rigid material and which receives the isolator portion 71. The outer structure 72 comprises a substantially cylindrical peripheral face 73 which tapers at its distal end portion. Furthermore, the outer structure 72 comprises a distal radial projection 74. As a result, there is formed a radial recess 75 at the distal end portion of the needle shield 70.

The needle shield 70 has to be removed by the user before the injection. However, as can be seen in figure 16, due to the presence of the skin contact member 50, the axial length of the needle shield 70 that is accessible beyond the distal end of the safety device 101 is reduced as compared to prior art safety devices. Thus, it may be difficult for the user to grip the needle shield 70. To solve this problem, injection system 100 may be provided with a cap 80 for increasing the surface area available for the user's fingers.

As shown in figures 14, 16 and 17, the cap 80 is removably mounted on the skin contact member collar 51 around the needle shield 70. For example, the cap 80 includes longitudinal proximal snap tabs 81 separated by slots 82, the snap tabs 81 cooperating with the proximal face 56 of the collar 51 in the mounted position of the cap 80.

The cap 80 further has inner tabs 83 which extend distally and form attaching means. Each inner tab 83 is provided with an inward projection 84. In the storage position, i.e. when the cap 80 is mounted on the skin contact member collar 51, the inward projection 84 of each inner tab 83 is in contact with the peripheral face 73 of the needle shield 70. Then, each inner tab 83 is elastically deflected outwards with respect to its rest position. In this storage position, the inner tabs 83 are disengaged from the needle shield 70.

When a user pulls the cap 80 distally to remove it, the snap tabs 81 are disengaged from the collar 51. In a first phase of the distal movement of the cap 80, the inward projections 84 of the inner tabs 83 slide distally along the peripheral face 73 of the needle shield 70, until they reach the recess 75. Because of their elasticity, the inner tabs 83 are then deflected inwards, towards their rest position, causing the inward projections 84 to engage the recess 75. The cap 80 and needle shield 70 are thus joined. As a result, in a second phase of the distal movement of the cap 80, the removal of the cap 80 from the safety device 101 entails the removal of the needle shield 70 from the syringe 10.

Other devices can be used to make the removal of the needle shield 70 easier for a user.

Although the skin contact member 50 has been described as being secured to the guard 30, in a variant of the previously described first embodiment, the skin contact member 50 could be secured to the body 20, provided enough radial space is provided to allow the movement of the guard 30 with respect to the body 20.

A second embodiment of the invention is now described with reference to figures 18 to 22.

The body 20 comprises a tubular portion 23 having a distal opening which forms the distal opening 25 of the body 20. The body 20 may be provided with finger flanges 21 at its proximal end portion. The body 20 may further comprise a proximal structure 85 which is arranged proximally from the finger flanges 21 and which has a distal face 86, the function of which will be explained later.

A tongue 87 is formed in the body tubular portion 23 by a slit 88 which has a distal portion arranged substantially orthogonally to the axis 105 and which forms an aperture 89. The body 20 preferably also comprises an inner rib 29.

The guard 30 preferably comprises a tubular portion 33 having a distal opening which forms the distal opening 35 of the guard 30. The guard 30 comprises a first ridge 91 which protrudes outwardly and is located at the guard distal end portion and a second ridge 92 which protrudes outwardly and is located proximally from the first ridge 91. The tubular portion 33 has a proximal part 39 which extends beyond the second ridge 92, in the proximal direction. The diameter of said proximal part 39 is smaller than the diameter of the part of the tubular portion 33 which extend distally from the second ridge 92.

The safety device 101 also comprises a sleeve 90 which has a cylindrical wall and a flange 93 which protrudes outwardly at the proximal end of the sleeve 90.

According to this second embodiment of the invention, the safety device 101 further comprises a skin contact member 50 which is arranged at and secured to the distal end portion of the body 20.

The skin contact member 50 comprises a collar 51 which has a distal face 52 that extends substantially in a plane orthogonal to the safety device axis 105. Said distal face 52 is configured to form a contact surface with the patient's skin, at the injection site. The collar 51 has an outer edge 53 and an inner edge 54 which defines a hole 55. The collar 51 can have the shape of a plate, and thus can have a proximal face 56 that also extends substantially in a plane orthogonal to the safety device axis 105.

The skin contact member 50 may further comprise a cylindrical sleeve 57 having an inner face 58, an outer face 59 and a distal face 61. The collar 51 may protrude radially outwardly from the sleeve distal end, the collar distal face 52 preferably being level with the sleeve distal face 61.

In order to ensure that the contact surface between the skin contact member 50 and the skin has a sufficient area, at least part of the collar 51 preferably extends radially beyond the tubular portion 23 of the body 20.

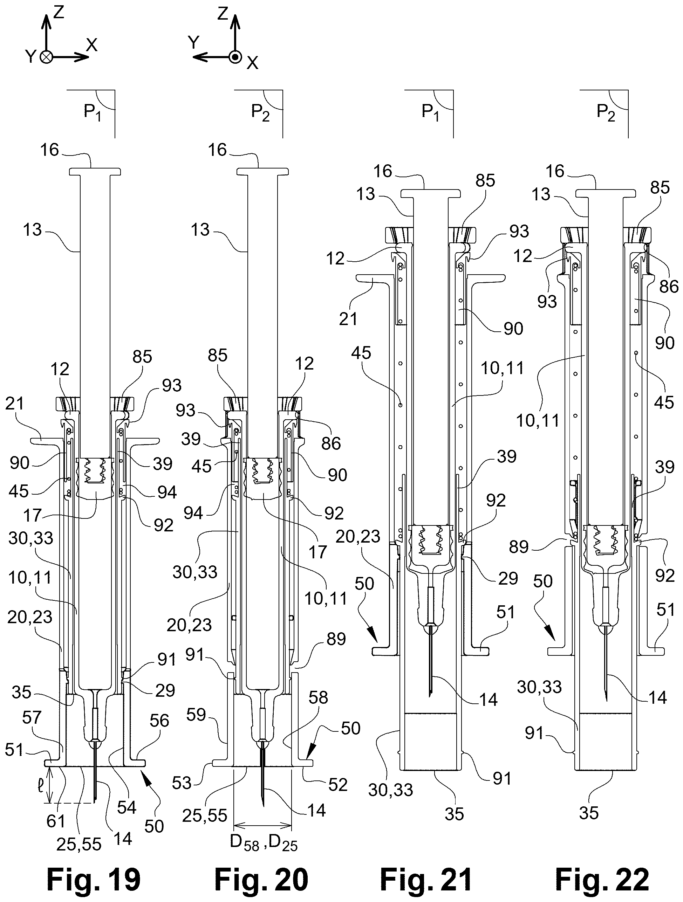

Preferably, as shown in figures 19 to 22, the cylindrical sleeve 57 forms the extension of the cylindrical shape of the body tubular portion 23. The skin contact member 50 is preferably integral with the body 20, for example molded as a single piece with the body 20. In particular, the cylindrical sleeve 57 may have an inner diameter D58 which is substantially identical to the diameter D25 of the distal opening 25 of the body 20, as shown in figure 20.

The body 20 may further comprise a slot 95 which extends longitudinally substantially from the aperture 89, in the distal direction, up to the distal opening 25 of the body 20 which is also the hole 55. The slot 95 preferably also extends radially in the collar 51 up to the outer edge 53. The slot 95 allows the body 20 radial deformation during the movement of the guard 30 from the injection position to the safety position.

As shown in figures 19 and 20, in the injection position, the guard 30 is mounted inside the body 20. Because of the reduced diameter of the proximal part 39 of the guard tubular portion 33, there is formed an annular gap 94 between said proximal part 39 and the body 20. The sleeve 90 is mounted between the guard 30 and the body 20 in said gap 94. Besides, the syringe 10 is inserted in the sleeve 90 and in the guard 30, proximally from the sleeve flange 93 and distally from the proximal structure 85 of the body 20.

A spring 45 is mounted in the body 20, in the gap 94. In the injection position, the spring 45 is compressed between the second ridge 92 of the guard 30 and the flange 93 of the sleeve 90. As a result, the spring 45 biases the flange 12 of the barrel 11 of the syringe 10 distally, in abutment against the distal face 86 of the proximal structure 85. The spring 45 also biases the guard 30 distally relative to the body 20. However, a distal movement of the guard 30 relative to the body 20 is prevented by the cooperation between the inner rib 29 of the body and the first ridge 91 of the guard 30.

In this injection position, the needle 14 passes through the hole 55, and extends beyond the distal end of the safety device 101, i.e. beyond the distal face 52 of the collar 51.

Similarly to the first embodiment, the invention makes it possible to set by design the needle exposure length I in the injection position, therefore ensuring an appropriate injection.

To perform injection, a user pushes the plunger rod 13 distally.

When injection has been completed, the stopper 17 is in abutment against the proximally-facing end face of the barrel 11. The user then removes the injection system 100 from the injection site, and keeps on exerting a distal force on the plunger rod 13? This tends to move the syringe 10 and the guard 30 distally. Under the action of a sufficient force, the first ridge 91 of the guard moves distally past the inner rib 29 of the body 20. Thus, the guard 30 is no longer maintained in position relative to the body 20, and the spring 45 causes the guard 30 to move distally towards its extended position beyond the body distal face, thereby covering the needle 14. The guard 30 is maintained in this position by means of the engagement of the second ridge 92 of the guard 30 in the aperture 89 of the body 20.

The user can then release the plunger rod 13. As a result, the spring 45 pushes the sleeve 90 distally, which in turn pushes the plunger rod proximal member 16 distally until said proximal member 16 comes in abutment against the distal face 86 of the proximal structure 85 of the body. During this step, the guard 30 does not move relative to the body 20. The safety device 101 is then in the safety position, as illustrated in figures 21 and 22.

Although the skin contact member 50 has been described as being secured to the body 20, in a variant of the previously described second embodiment, the skin contact member 50 could be secured to the guard 30, provided enough radial space is provided to allow the movement of the guard 30 with respect to the body 20.

In a non-described variant, the safety device 101 may be designed such that the guard needs to be moved manually to the safety position.

It is to be understood that the present invention is not limited to the embodiments described above and illustrated in the drawings; rather, the skilled person will recognize that many changes and modifications may be made within the scope of the appended claims.

## Claims

1. A safety device (101) for a syringe (10) having a needle (14), the safety device (101) having an axis (105) and comprising:
- a body (20) configured to receive the syringe (10), the body (20) having a distal opening (25);
- a guard (30) having a distal opening (35), the guard (30) being movable relative to the body (20) between an injection position in which the needle (14) extends beyond the distal end of the guard (30), and a safety position in which the guard (30) covers the needle (14);
**characterized in that** it further comprises a skin contact member (50) which is arranged at and secured to the distal end portion of one element among the body (20) and the guard (30), said element being called the support element, said skin contact member (50) comprising a collar (51) having a distal face (52) that extends substantially in a plane orthogonal to the safety device axis (105), said distal face (52) being configured to form a contact surface with the skin and to be located proximally from the needle tip in the injection position, the collar (51) having an outer edge (53) and an inner edge (54) defining a hole (55) for the needle (14) to pass through.

2. The safety device according to claim 1, wherein the collar outer edge (53) is at least partially located radially further from the axis (105) than the support element outer face.

3. The safety device according to claim 1 or claim 2, wherein the axial length of the skin contact member (50) is comprised between 4 and 30 %, or 5 and 25 %, or 5 and 10 %, of the axial length of the support element.

4. The safety device according to any one of claims 1 to 3, wherein the collar (51) has the shape of a plate and has a proximal face (56) that extends substantially in a plane orthogonal to the safety device axis (105).

5. The safety device according to any one of claims 1 to 4, wherein the skin contact member (50) comprises a cylindrical sleeve (57) having an inner diameter (D58) which is preferably substantially identical to the diameter (D25, D35) of the support element distal opening (25, 35), the proximal face of the cylindrical sleeve (57) being adjacent or in contact with the support element distal face, wherein the collar (51) protrudes radially outwardly from the sleeve distal end.

6. The safety device according to any one of claims 1 to 5, wherein the skin contact member (50) is a part separate from the support element and secured to the distal end portion of said support element.

7. The safety device according to claim 6, wherein the skin contact member (50) is secured to the support element distal end face.

8. The safety device according to claim 6, wherein the skin contact member (50) comprises a proximal casing (65) having a proximal aperture (67), the distal end portion of the support element being received in said proximal casing (65) and secured thereto.

9. The safety device according to one of claims 6 to 8, further comprising an extended finger flange secured to a finger flange of the guard (30).

10. The safety device according to any one of claims 1 to 5, wherein the skin contact member (50) is integral with the support element, for example molded as a single piece with said support element.

11. The safety device according to claims 5 and 10, with the support element being the body (20), wherein the body (20) has a cylindrical shape and the cylindrical sleeve (27) forms the extension of the cylindrical shape of the body (20).

12. The safety device according to any one of claims 1 to 10, wherein the support element is the guard (30), the guard (30) being arranged outside the body (20) and receiving the body (20).

13. The safety device according to any one of claims 1 to 12, wherein the skin contact member (50) comprises at least one protrusion (63) which protrudes distally from the distal face (52) of the collar (51) and which is configured to improve the contact between the collar (51) and the skin.

14. The safety device according to any one of claims 1 to 13, wherein the distal face (52) of the collar (51) of the skin contact member (50) is at least partially covered with an adhesive (62).

15. An injection system (100) comprising a safety device (101) according to any one of the preceding claims and a syringe (10) received in the body (20), the syringe (10) having a needle (14), wherein, in the injection position, the needle tip is located proximally from the collar distal face (52).

16. The injection system according to claim 15, further comprising a needle shield (70) attached to the syringe (10) for covering the needle (14) in the storage position, wherein the injection system (100) further comprises a cap (80) which is removably mounted on the skin contact member collar (51) around the needle shield (70), the cap (80) having inner attaching means (83) which are disengaged from the needle shield (70) in the storage position and which can move to engage the needle shield (70) in a first phase of the removal of the cap (80) from the collar (51), so that in a second phase, the removal of the cap (80) from the collar (51) entails the removal of the needle shield (70) from the syringe (10).
